# EUROPEAN PATENT APPLICATION

(11) **EP 4 134 056 A1**
(43) Date of publication of application: **15.02.2023**
(21) Application number: 21191372.8
(22) Date of filing: 13.08.2021
(51) Int. Cl.: A61G 13/04, A61B 34/00, A61B 34/30

(54) **MEDICAL ROBOTIC ARM FOR USE WITH A MOVEABLE SURGICAL TABLE**

(71) Applicant: TRUMPF Medizin Systeme GmbH + Co. KG, 07318 Saalfeld (DE)
(72) Inventor: DALBERT, Heinz-Hermann, 07318 Saalfeld (DE)
(74) Representative: Loustalan, Paul William

(57) **Abstract**

A medical robotic arm for use with a moveable surgical table. The medical robotic arm comprises an overhead mount for mounting the medical robotic arm above the moveable surgical table; a mechanical interface connectable to one or more devices for use in minimally invasive surgery; and a positioning assembly. The positioning assembly comprises a first end comprising a first motorised joint connected to the mechanical interface, a second end comprising a second motorised joint connected to the overhead mount, and a middle portion connecting the first end to the second end. The medical robotic arm further comprises a first position detector configured to monitor the position of the first motorised joint; a second position detector configured to monitor the position of the second motorised joint; and a controller. The controller is configured to maintain the position of the mechanical interface relative to the moveable surgical table based on one or more control signals.

## Description

### TECHNICAL FIELD

The present disclosure relates to a medical robotic arm for use with a moveable surgical table; a system comprising the medical robotic arm and a moveable surgical table; and a method of controlling the medical robotic arm. In particular, the disclosure relates to a medical robotic arm for use in minimally invasive surgery.

### BACKGROUND

The use of medical robotic arms during surgery is becoming more widespread. A medical robotic arm can assist a surgeon or team of surgeons during a surgical procedure. For example, a medical robotic arm can support a medical device or surgical robot that is used to perform the surgery at a device end of the medical robotic arm. Medical robotic arms may also support other devices such as torches or cameras.

Medical robotic arms have particular application in minimally invasive surgery in which the surgeon minimises the size and number of cuts made into the patient during the surgical procedure. This is because a medical robotic arm is typically able to provide more accurate and steady control than could be achieved by a person. Medical robotic arms also improve accessibility during surgery. One application of medical robotic arms is in remote surgery (telesurgery).

It is important that medical robotic arms used in surgery, and in particular in minimally invasive surgery, are correctly positioned with respect to the surgical table on which the patient lies during the procedure so that a device supported by the device end of the medical robotic arm is held in a suitable position relative to the patient. However, the surgical table is often moveable. A surgeon may move the table during surgery to reposition the patient, for example. A corresponding adjustment to the medical robotic arm is then needed if its device end is to remain in the same position relative to the patient. In known systems comprising robotic arms, this adjustment can be cumbersome and is a distraction for the surgeon from the ongoing surgical procedure. Furthermore, in certain operations, the device supported by the device end should be in contact with the patient. Said contact cannot easily be maintained during the repositioning of the medical robotic arm and table.

It would be desirable to provide a medical robotic arm which is simpler and easier to operate in conjunction with a moveable surgical table. It would also be desirable to be able to simply maintain contact between the device end of the medical robotic arm and / or device supported by the device end and the patient during repositioning.

Known medical robotic arms are typically positioned at the side of a moveable table, either mounted to a side rail of the surgical table or to a cart adjacent to the surgical table. This has several disadvantages. Precise positioning of the medical robotic arm is required before the surgery begins which is time consuming. This is a particular problem given the importance of minimising dead time between operations in an operating theatre. Furthermore, a side mounting is typically not stable enough for accurate control of the device end. In particular, the side rail of a surgical table is often not rigid enough to provide a satisfactory single mounting point. Furthermore, medical robotic arms can be bulky and so, when positioned at the side of a moveable table, can intrude in the surgeons' workspace. The power and control cables for the medical robotic arm can also get in the way. Again, it would be desirable to provide a medical robotic arm that is simpler and easier to operate, in particular that is simpler and easier to set up prior to surgery. It would be desirable to provide a robotic arm that can be securely mounted and that minimally intrudes on the surgeon's workspace.

It would also be desirable to provide a medical robotic arm that solves the above identified problems, while also incorporating suitable safety features to prevent harm to patients.

### SUMMARY OF THE DISCLOSURE

The present disclosure provides a medical robotic arm, a system comprising the medical robotic arm and a method of controlling the medical robotic arm as defined in the appended claims, to which reference should now be made.

According to a first aspect of the present disclosure, there is provided a medical robotic arm for use with a moveable surgical table. The medical robotic arm comprises an overhead mount for mounting the medical robotic arm above the moveable surgical table. The medical robotic arm also comprises a mechanical interface connectable to one or more devices for use in minimally invasive surgery. The medical robotic arm also comprises a positioning assembly comprising a first end comprising a first motorised joint connected to the mechanical interface, a second end comprising a second motorised joint connected to the overhead mount, and a middle portion connecting the first end to the second end. The medical robotic arm also comprises a first position detector configured to monitor the position of the first motorised joint and a second position detector configured to monitor the position of the second motorised joint. The medical robotic arm further comprises a controller configured to maintain the position of the mechanical interface relative to the moveable surgical table based on one or more control signals.

An overhead mount advantageously provides a stable mounting point. The overhead mount may be fixed to the ceiling, for example the ceiling of an operating theatre, above the moveable surgical table.

The position of the overhead mount at the second end of the positioning assembly may typically be fixed during use of the medical robotic arm. The positioning assembly, and particularly the first and second motorised joints of the positioning assembly, may therefore advantageously be used to move the first end and the mechanical interface relative to the overhead mount.

Mounting the medical robotic arm overhead may advantageously reduce the amount that the medical robotic arm intrudes into a working area of the surgeon. Any power and control cables associated with the medical robotic arm may be kept out of the way, for example above the working area of the surgeon. The bulk of the robotic arm may also be above the table and the working area of the surgeon.

Furthermore, mounting the medical robotic arm overhead advantageously simplifies set up of the medical robotic arm for surgery. If the medical robotic arm is needed, it may be lowered from the ceiling and positioned over the moveable surgical table as desired. If the medical robotic arm is not needed, it can be raised and stored overhead. This may advantageously remove the need for a user to disconnect or reconnect the medical robotic arm between procedures reducing set up times and reducing the need for users to handle heavy equipment. A reduction in set up time may advantageously reduce dead time in the operating theatre.

The controller may receive signals from the first and second position detectors.

The controller may advantageously be configured to use the monitored positions of the first and second motorised joints by the first and second position detectors, respectively, in order to maintain the position of the mechanical interface relative to the moveable surgical table.

The monitoring of the position of the first and second motorised joints by the first and second position detectors advantageously may allow the position of the mechanical interface to be determined by the controller. The absolute position of the mechanical interface may be determined by the controller.

To determine the position of the mechanical interface, the controller may receive signals from the first and second position detectors, the signals relating to the position of the first and second motorised joints. The signals may relate to the angle of each of the first and second joints. The controller may be configured to determine the position of the mechanical interface with respect to the overhead mount based on the received signals. For example, the controller may store in a memory dimensions of features of the robotic arm such as the first and second ends and the middle portion. The controller may be configured to use information relating to the angle of the joints and the stored dimensions to calculate the position of the mechanical interface.

Preferably, if the mechanical interface is moved from a first position to a second position, the controller may calculate the absolute position of the mechanical interface in both the first and second positions.

Alternatively, the first and second position detectors may monitor the position of the first and second motorised joints only while the motorised joints are moving. In this mode of operation, the controller may not calculate an absolute position of the mechanical interface.

The controller being configured to maintain the position of the mechanical interface relative to the moveable table may advantageously mean that there is no need for the surgeon to independently move the surgical table and then adjust the robotic arm so that the device for use in minimally invasive surgery remains fixed relative to the patient on the surgical table. The relative position of the mechanical interface to the moveable surgical table is advantageously maintained automatically.

As used herein, "device for use in minimally invasive surgery" is intended to mean any device that can be used in in minimally invasive surgery. This includes medical devices and surgical robots as well as a torch or a camera. The device may itself comprise moveable or actuatable parts which can be controlled separately from the medical robotic arm. For example, the device may be an articulated surgical instrument. The medical robotic arm may support the articulated surgical instrument so that the end of the instrument that is connected to the mechanical interface is maintained in a particular position relative to the moveable surgical table. However, because the surgical instrument is articulated, it may be moveable relative to the mechanical interface so that the surgeon can perform a surgical procedure on a patient on the moveable table using the surgical instrument without moving the medical robotic arm.

The mechanical interface is connectable to one or more devices for use in minimally invasive surgery. Thus, the mechanical interface may be connected to or disconnected from a first device for use in minimally invasive surgery. The mechanical interface may be connectable to more than one device for use in minimally invasive surgery. Thus, the mechanical interface may be connected to or disconnected from a second device as well as the first device, for example. This advantageously allows the medical robotic arm to be used in a greater range of minimally invasive surgical procedures than if the mechanical interface was connectable to a single device.

The mechanical interface and/or a device for performing minimally invasive surgery connected to the mechanical interface may preferably be in contact with a patient lying on the moveable surgical table during use of the medical robotic arm when the mechanical interface and moveable surgical table are in a first position. The controller being configured to maintain the position of the mechanical interface relative to the moveable table may advantageously mean that said contact is maintained in both the first and second positions of the mechanical interface and moveable surgical table.

The medical robotic arm may comprise a receiver for receiving an inbound control signal. The controller may be configured to control the positioning assembly to move the mechanical interface based on received inbound control signals. The inbound control signals may be related to the position of the moveable surgical table.

As used herein, "inbound" in the context of a control signal means a signal received or receivable by a receiver of the medical robotic arm. This is the case even when the generation of the inbound control signal is described in relation to an external source. For example, a signal generated by the moveable surgical table and transmitted to the medical robotic arm is still referred to as an inbound control signal.

As used herein, "outbound" in the context of a control signal mean a signal transmitted by a transmitter of the medical robotic arm.

The inbound control signal may be related to the moveable surgical table being moved from a first position to a second position. For example, the inbound control signal may be related to the moveable surgical table being moved "upwards" ten millimetres.

As used herein, "upwards" means in a direction towards the overhead mount and "downwards" means away from the overhead mount. It should be understood that, while an example of moving upwards by ten millimetres has been used herein, the moveable surgical table and/or mechanical interface of the medical robotic arm may be moveable in other directions by other amounts.

The controller may be configured to control the positioning assembly to move the mechanical interface with respect to the overhead mount from a first position to a second position based on the inbound control signal. The controller may be configured to calculate an amount or angle to move at least one of the first and second motorised joints based on the inbound control signal.

The position of the mechanical interface relative to the moveable surgical table may be the same when the moveable surgical table and the mechanical interface are in their respective first positions as when the moveable surgical table and the mechanical interface are in their respective second positions. In this way, the medical robotic arm automatically maintains the position of the mechanical interface relative to the moveable surgical table. As an example, if the inbound control signal is related to the moveable surgical table being moved "upwards" ten millimetres, the second position of the mechanical interface would be ten millimeters above the first position.

Preferably, the controller may be configured to determine the first position of the mechanical interface before moving the positioning assembly based on the inbound control system. The controller may then be configured to determine the second position for the mechanical interface based on the inbound control signal and the determined first position.

Alternatively, the controller may be configured to directly convert the inbound control signal into an amount or an angle to move at least one of the first and second motorised joints. In this mode of operation, the controller may not receive signals relating to the initial position of the first and second motorised joints and may not calculate an absolute position of the mechanical interface.

The controller may comprise the receiver.

The receiver may be configured for receiving inbound control signals from the moveable surgical table. The inbound control signal may be generated by the moveable surgical table in response to the surgeon inputting a command to a user interface of the moveable surgical table. The command may be to move the table. Alternatively, the inbound control signal may be generated by the surgeon manually repositioning the moveable surgical table. In this case, the moveable surgical table may comprise position detectors configured to monitor a change in position of the moveable surgical table. A controller of the moveable surgical table may translate a detected change in position of the moveable surgical table into the inbound control signal.

The receiver may be configured for receiving inbound control signals from some other transmitter. For example, the transmitter may be part of a telesurgery system allowing communication between the medical robotic arm and a remote surgeon.

The receiver may be a wireless receiver. The wireless receiver may be configured for receiving wireless inbound control signals transmitted from the moveable surgical table. The wireless receiver may be a Bluetooth receiver or a 5G receiver. Bluetooth or 5G advantageously have suitably low latency to avoid noticeable delays between transmission of the inbound control signal and movement of the medical robotic arm. Transferring control signals over Bluetooth or 5G may also advantageously be secure.

The medical robotic arm may alternatively or additionally comprise a transmitter for transmitting an outbound control signal generated by the controller of the medical robotic arm. The outbound control signal may be operable by the moveable surgical table to move the surgical table. Based on the outbound control signal, the moveable surgical table may advantageously be configured so as to move to maintain its position relative to the mechanical interface.

The positioning assembly may be configured such that a user may physically move the mechanical interface from a first position to a second position. For example, the first and second motorised joints may be configured to be moveable when an external force is applied by the user. In this way, the user may manually manipulate the position of the mechanical interface with respect to the overhead mount.

The controller may be configured to generate the outbound control signal in response to the mechanical interface being moved by a user of the medical robotic arm from a first position to a second position. This may advantageously mean that, if a user manually moves the mechanical interface from the first and second position, a corresponding outbound control signal may be transmitted to the moveable surgical table. For example, if the controller determines that the mechanical interface has been moved upwards by ten millimeters, the outbound control signal may comprise a command for the moveable surgical table to move ten millimeters upwards also. The moveable surgical table may be configured to then move in response to the outbound control signal to automatically maintain the position of the moveable surgical table relative to the mechanical interface.

The controller may be configured to calculate the outbound control signal based on positional information of the first and second motorised joints received from the first and second position detectors respectively. The outbound control signal may be generated based on a detected change of position of at least one of the first and second motorised joints.

As described above, the controller may preferably be configured to determine the first position of the mechanical interface based on positional information of the first and second motorised joints. The controller may also be configured to determine the position of the mechanical interface after it has been moved to the second position based on the positional information. The controller may be configured to calculate the outbound control signal based on the difference between the first position and the second position.

Alternatively, the first and second position detectors may monitor the position of the first and second motorised joints only while the mechanical interface is moved from the first position to the second position. The controller may be configured to determine the amount or angle that each of the first and second motorised joints are moved while the mechanical interface is moved from the first position to the second position, if at all. The controller may be configured to directly convert the amount or angle of movement of each of the first and second motorised joints into the outbound control signal.

The medical robotic arm may comprise a user interface connected to the controller. Alternatively or additionally to the generation of the control signal being in response to the mechanical interface being moved by a user of the medical robotic arm from a first position to a second position, the controller may be configured to generate a control signal in response to a user input on the user interface. The transmitter may be configured to transmit the generated control signal as the outbound control signal. The controller may also be configured to control the positioning assembly to move the mechanical interface based on the generated control signal. Thus, a user of the medical robotic arm may advantageously be able to control the position of the mechanical interface by inputting commands to the user interface. At the same time, the generated control signal may advantageously be transmitted to the moveable surgical table which is configured to move in a corresponding way in response to receiving that signal. In this way, the position of the mechanical interface relative to the moveable surgical table is maintained automatically as the medical robotic arm moves from a first position to a second position.

The user interface may comprise buttons or switches corresponding to each direction in which the medical robotic arm can move. For example, upwards and downwards.

The transmitter may be a wireless transmitter. The wireless transmitter may be configured for transmitting wireless outbound control signals to be received by the moveable surgical table. The wireless transmitter may be a Bluetooth transmitter or 5G transmitter. Bluetooth and 5G advantageously have suitably low latency to avoid noticeable delays between transmission of the outbound control signal and movement of the moveable surgical table. Transferring control signals over Bluetooth or 5G may also advantageously be secure.

The one or more control signals may be encrypted. An encrypted signal is advantageously more secure.

The controller may be configured to control the positioning assembly to move the mechanical interface based on the control signal operated on by the moveable surgical table. This may advantageously be simpler than providing control signals tailored to the medical the robotic arm or the moveable surgical. In this way, the same control signal can advantageously be used with medical robotic arms and/or moveable surgical tables of different dimensions. The control signal may simply be provided as an instruction to move the mechanical interface and moveable surgical table in a certain way, for example upwards ten millimeters. The controller of the robotic medical arm may be configured to convert the control signal into specific instructions for controlling the positioning assembly so as to move the mechanical interface as per the control signal. This may comprise the controller calculating the number of degrees to move at least one of the motorised joints based on dimensions of the robotic arm. The dimensions of the robotic arm may be stored in a memory of the controller. A controller of the moveable surgical table may also be configured to convert the control signal into specific instructions for moving the surgical table.

Preferably, the controller may be configured to control the positioning assembly to move the mechanical interface based on an identical control signal to that operated on by the moveable table.

The control signals for the controller of the medical robotic arm and the moveable surgical table may be synchronised. Alternatively or additionally, the controller of the medical robotic arm may be configured to move the medical robotic arm simultaneously with the moveable surgical table, in a synchronised way. This may be particularly advantageous when the mechanical interface or devices connected to the mechanical interface are to be maintained in contact with a patient during use. That contact may be maintained automatically throughout the repositioning of the medical robotic arm and the moveable surgical table. When the control signal is based on a user manually moving the medical robotic arm, or manually moving the moveable surgical table, this may be achieved with a continuous stream of control signals each representing a small part of that movement. In other words, the distance between the first position and the second position may be relatively small. Simultaneous movement of the medical robotic arm and the moveable surgical table may require a well aligned system and for the spatial positioning of the mechanical interface and the moveable surgical table to be well known. The latter may be achieved by using encoders to monitor the absolute position of the first and second motorised joints, as described below.

It has been described how the medical robotic arm may comprise a receiver for receiving an inbound control signal related to the position of the moveable surgical table. It has also been described how the medical robotic arm may comprise a transmitter for transmitting an outbound control signal generated by the controller of the medical robotic arm, the outbound control signal being operable by the moveable surgical table. It should be understood that the controller being configured to maintain the position of the mechanical interface relative to the moveable surgical table may be based on a combination of both inbound control signals and outbound control signals. For example, an outbound control signal may be transmitted to the moveable surgical table causing the moveable surgical table to move. However, while the moveable surgical table is moving based on that outbound signal, the moveable surgical table may periodically generate an inbound control signal that is received by the medical robotic arm. Based on the periodic inbound control signals, the controller of medical robotic arm may be configured to monitor the position of the moveable surgical table while it moves. The reverse arrangement may also be possible where a controller of the moveable surgical table may be configured to monitor the position of the medical robotic arm while the medical robotic arm is moving based on an inbound control signal. This periodic position monitoring may be particularly advantageous when the medical robotic arm and movable surgical table are intended to move simultaneously. It may advantageously mean that one of the medical robotic arm or moveable surgical table stopping or rejecting a desired movement, perhaps because of a collision, can be reported to the other of the medical robotic arm or moveable surgical table.

The overhead mount may be a ceiling mount for mounting the medical robotic arm to a ceiling above the moveable surgical table. In other words, the medical robotic arm may be directly mounted to the ceiling. Alternatively, the overhead mount may be fixed to an intermediate fixture which itself is connected to the ceiling. In either case, mounting the medical robotic arm directly or indirectly to the ceiling has the advantage of providing a secure mounting.

The first motorised joint may comprise a first stepper motor. A stepper motor may be a motor in which a full rotation is divided into a number of equal, discrete, steps. The first motorised joint comprising a first stepper motor may be advantageous because it is straightforward to command a stepper motor to accurately rotate a specific amount equal to a integer number of steps. When not in motion, a stepper motor also advantageously may hold its position in a specific step which means that the mechanical interface is held in position by the stepper motor. The first stepper motor may be commandable to rotate a discrete number of steps by the controller.

The first position detector may comprise the first stepper motor.

Preferably, the medical robotic arm may comprise an encoder configured to monitor the position the first stepper motor. The encoder may be configured to monitor the absolute position of the first stepper motor. The encoder may monitor the position of the first stepper motor when the first stepper motor is commanded to move by the controller. The encoder may additionally or alternatively monitor the position of the first stepper motor while a user manually moves the mechanical interface. The first stepper motor may comprise the encoder. The encoder may be connected to the controller. The encoder may be a single-turn absolute encoder.

Using an encoder to monitor the position of the first stepper motor may be advantageous because the controller is not required to continuously track the number of steps moved by the first stepper motor in order to determine the position of the mechanical interface. This information may instead be encoded in a signal received at the controller from the encoder. Furthermore, using the absolute position determined by the encoder means that, if the first stepper motor misses a step, positional information received at the controller from the encoder may still advantageously represent the true position of the first stepper motor. Furthermore, following power loss to the controller, the position of the first stepper motor can simply be re-obtained from the encoder.

The second motorised joint may comprise a second stepper motor that also acts as the second position detector. The features and advantages of the second stepper motor may be the same as the first stepper motor. Furthermore, the medical robotic arm may comprise a second encoder configured to monitor the position of the second stepper motor.

Alternatively, because the first stepper motor is moveable only in discrete steps, the position of the first stepper motor may be monitored simply by tracking the number of steps that the first stepper motor moves. In this way, the position of the first stepper motor is known.

The controller may be configured to track the number of steps that the first stepper motor moves. This may include tracking the number of steps that the first stepper motor is commanded to move by the controller.

The axis of rotation of the first motorised joint may be parallel to the axis of rotation of the second motorised joint. The mechanical interface may thus be positionable by the positioning assembly anywhere within a plane that is perpendicular to the axis of rotation of the first and second motorised joints.

Perpendicular X and Y axes may lie in the plane, the axes defining X and Y directions respectively. The mechanical interface may be moveable in both the X and Y direction by the first and second motorised joints having parallel axes of rotation.

The medical robotic arm may comprise a second positioning assembly configured to move the mechanical interface in a direction out of the plane that is perpendicular to the axis of rotation of the first and second motorised joints. This may advantageously allow for control of the mechanical interface in a Z direction that is perpendicular to both the X and Y direction.

The second positioning assembly may comprise a rotatable joint having an axis of rotation that is not parallel to the axis rotation of the first and second motorised joints. The rotatable joint may have an axis of rotation that is perpendicular to the axis of rotation of the first and second motorised joints. The overhead mount may comprise the second positioning assembly. Alternatively, the positioning assembly may comprise the second positioning assembly. The second end of the positioning assembly may comprise the second positioning assembly. In that case, the second positioning assembly may preferably be positioned between the connection to the overhead mount and the second motorised joint.

The medical robotic arm may further comprise a socket for receiving a wired connection for receiving and/or sending control signals to the moveable table. The wired connection may be in addition to the wireless receiver or transmitter described above. The wired connection may advantageously act as a back-up means of transferring control signals between the medical robotic arm and the moveable table in case the wireless transmission fails.

The medical robotic arm may comprise other safety features, as follows.

The controller may be configured to prevent movement of the first and second motorised joints in the event the first and/or second position detector fail. The failure may be detected by the controller. Failure may mean that first and/or second position detector stop monitoring the position of the respective motorised joints. Failure may also mean the controller stopping receiving signals from the first and/or second position detectors or the control signals being corrupted.

The controller may be configured to receive positional information about the first and second motorised joint from a back-up position detector. The back-up position detector may be separate to the medical robotic arm. Alternatively, the medical robotic arm may comprise the back-up position detector. The back-up position detector may be a 3D depth camera. The controller may use the signals received from the 3D depth camera to determine the absolute position of the mechanical interface and, from the absolute position of the mechanical interface, the controller may be configured to calculate the position of the first and/or second motorised joints. The back-up position detector thus advantageously provides a means to determine the position of the first and second motorised joint even if there is a problem with the first and/or second position detector.

As described above, the controller may be configured to control the positioning assembly to move the mechanical interface based on received inbound control signals. The controller may be configured to detect an external force applied the medical robotic arm while it is moving. For example, if the first and second motorised joints comprise stepper motors with first and second encoders, a user applying a force on the medical arm may prevent at least one of the stepper motors from moving a step, as commanded by the controller. The mismatch between the expected change of position of the first and/or stepper motor and actual change of position, or lack thereof, detected by the first and/or second encoder may be detected by the controller. The controller may stop movement of the mechanical interface in response to such a mismatch. This may be an advantageous safety feature. A surgeon using the medical robotic arm can apply an external force to stop unwanted movement of the medical robotic arm. This may be because he moved the moveable surgical table but did not intend to the medical robotic arm to follow. An external force may also be applied if the medical robotic arm collides with another object or a wall or ceiling. The medical robotic arm may therefore cease movement following such a collision.

The middle portion of the positioning assembly may comprise a third motorised joint. A first end of the middle portion may connect the first motorised joint to the third motorised joint. A second end of the middle portion may connect the second motorised joint to the third motorised joint. The axis of rotation of the first motorised joint may be parallel to the axis of rotation of the first and second motorised joints. The provision of such a third motorised joint may advantageously provide more ways that the mechanical interface can be moved within a plane that is perpendicular to the axis of rotation of the first, second and third motorised joints.

The medical robotic arm may comprise a third position detector configured to monitor the position of the third motorised joint. The controller may be configured to control the third motorised joint similarly to as described above in relation to the first and second motorised joints.

The third motorised joint may comprise a third stepper motor that also acts as the third position detector. The third motorised joint may comprise a third encoder.

According to a second aspect of the present disclosure, there is provided a system comprising the medical robotic arm of the first aspect and a moveable surgical table below the medical robotic arm. The moveable surgical table comprises a surgical table, a table positioning assembly and a controller configured to maintain the position of the mechanical interface of the medical robotic arm relative to the surgical table based on one or more control signals.

The moveable surgical table may further comprise one or more position detectors configured to monitor the position of the surgical table. The surgical table may be moveable in at least an X and a Y direction. The X and Y direction may lie in the same plane as the plane defined by the parallel axes of rotation of the first and second motorised joints of the medical robotic arm. The moveable surgical table may comprise a first position detector to determine the position of the moveable table in the X axis and a second position detector to determine the position of the moveable table in the Y axis.

The surgical table may tilt such that the head of a patient lying on the table is raised while the feet are lowered, or vice versa. The surgical table may be configured to twist left of right such that one arm of a patient lying on the table is raised while the other is lowered, or vice versa. In both cases, the medical robotic arm may be required to move the mechanical in a plane perpendicular to the axis of rotation of the first and second motorised joints in order to maintain the position of the mechanical interface relative to the moveable surgical table. The moveable surgical table may comprise further position detectors configured to monitor at least one of the tilt and twist of the surgical table.

The surgical table may be configured to tilt over a range of up to 45 degrees. The surgical table may be configured to twist over a range of up to 35 degrees. The positioning assembly of the medical robotic arm may be configured such that the range of movement of the mechanical interface is suitable to maintain the position of the mechanical interface relative to the moveable table across the full range of movement of the moveable table.

The moveable surgical table may comprise a transmitter for transmitting a control signal, the control signal being an inbound control signal for the medical robotic arm. The inbound control signal may be received by the receiver of the medical robotic arm. The inbound control signal may be generated by the controller of the moveable surgical table. Based on the outbound control signal, the medical robotic arm may advantageously be configured so as to move to maintain its position relative to the moveable surgical table, as described in relation to the first aspect.

The surgical table may be configured such that a user may physically reposition the surgical table from a first position to a second position.

The controller of the moveable surgical table may be configured to generate the inbound control signal in response to the moveable surgical table being moved by a user from a first position to a second position. This may advantageously mean that, if a user manually moves the surgical table from the first and second position, a corresponding inbound control signal may be transmitted to the medical robotic arm. For example, if the controller of the moveable surgical table determines that the surgical table has been moved upwards by ten millimeters, the inbound control signal may comprise a command for the mechanical interface of the medical robotic arm to move ten millimeters upwards also. The medical robotic arm may be configured to then move in response to the inbound control signal, as described in the first aspect, to automatically maintain the position of the moveable surgical table relative to the mechanical interface.

The controller of the moveable surgical table may be configured to calculate the outbound control signal based on positional information of the moveable table received from the one or more position detectors of the moveable surgical table.

The moveable surgical table may comprise a user interface. The user interface may be connected to the controller. Alternatively or additionally to the generation of the inbound control signal being in response to the mechanical interface being moved by a user of the surgical table from a first position to a second position, the controller may be configured to generate a control signal in response to a user input on the user interface. The transmitter of the moveable surgical table may be configured to transmit the generated control signal as the inbound control signal. The controller of the moveable surgical table may also be configured to control the positioning assembly to move the surgical table based on the generated control signal. Thus, a user of the system may advantageously be able to control the position of the moveable surgical table by inputting commands to the user interface. At the same time, the generated control signal may advantageously be transmitted to the medical robotic arm which is configured to move in a corresponding way in response to receiving that signal. In this way, the position of the mechanical interface relative to the moveable surgical table is maintained automatically as the surgical table moves from a first position to a second position.

The user interface may comprise buttons or switches corresponding to each direction in which the surgical table is moveable. For example, upwards and downwards.

The transmitter of the moveable surgical table may be a wireless transmitter. The wireless transmitter may be configured for transmitting wireless inbound control signals to be received by the medical robotic arm. The wireless transmitter may be a Bluetooth transmitter or 5G transmitter.

The moveable surgical table may alternatively or additionally comprise a receiver for receiving an outbound control signal. The controller may be configured to control the positioning assembly to move the mechanical interface based on received outbound control signals. The outbound control signals may be related to the position of the medical robotic arm. In particular, the outbound control signals may be related to the position of the mechanical interface of the medical robotic arm. For example, the outbound control signal may be related to the mechanical interface being moved "upwards" ten millimetres. The controller of the moveable surgical table may be configured to calculate an amount and direct to move the moveable surgical table based on the outbound control signal. The controller of the moveable surgical table may be configured to move the surgical table from a first position to a second position. The position of the mechanical interface relative to the moveable surgical table may be the same when the moveable surgical table and the mechanical interface are in their respective first positions as when the moveable surgical table and the mechanical interface are in their respective the second positions. In this way, the position of the surgical table relative to the mechanical interface may advantageously be automatically maintained.

The receiver of the moveable surgical table may be configured for receiving outbound control signals from the medical robotic arm. The outbound signal may be generated by the controller of medical robotic arm as described in first aspect.

The receiver may be configured for receiving outbound control signals from some other transmitter. For example, the transmitter may be part of a telesurgery system allowing communication between the medical robotic arm and a remote surgeon.

The receiver of the moveable surgical table may be a wireless receiver. The wireless receiver may be configured for receiving wireless inbound control signals transmitted from the moveable surgical table. The wireless receiver may be a Bluetooth receiver or a 5G receiver.

The controller configured to maintain the position of the mechanical interface relative to the moveable surgical table based on one or more control signals, of the first and second aspects, may comprise at least one processor, and memory encoding instructions which, when executed by the at least one processor, cause the at least one processor to be configured to carry out the control steps described herein.

According to a third aspect of the present disclosure, there is provided a method of controlling a medical robotic arm for use with a moveable surgical table, the medical robotic arm comprising an overhead mount for mounting the medical robotic arm above the moveable surgical table; a mechanical interface connectable to one or more devices for use in minimally invasive surgery; and a positioning assembly comprising a first end comprising a first motorised joint connected to the mechanical interface, a second end comprising a second motorised joint connected to the overhead mount, and a middle portion connecting the first end to the second end; the method comprising:
monitoring the position of the first motorised joint with a first position detector and monitoring the position of the second motorised joint with a second position detector; and
maintaining the position of a mechanical interface relative to the moveable surgical table based on one or more control signals.

The medical robotic arm controlled in the method may be a medical robotic arm according to the first aspect.

The step of maintain the position of a mechanical interface relative to the moveable surgical table may comprise at least one of the following steps:
receiving an inbound control signal related to the position of moveable surgical table and controlling the positioning assembly to move the mechanical interface based on the inbound control signal; or
generating an outbound control signal in response to the mechanical interface being moved by a user of the medical robotic arm from a first position to a second position and transmitting that outbound control signal to the moveable surgical table; or
generating a control signal in response to a user input on a user interface of the surgical robotic arm; controlling the positioning assembly to move the mechanical interface based on the generated control signal and transmitting the generated signal to the moveable surgical table as an outbound signal.

The advantages of maintaining the position of the mechanical interface relative to the moveable table have been discussed above, in relation to the first aspect.

The step of receiving an inbound control signal may comprise receiving the inbound signal at a receiver of the medical robotic arm.

The step of controlling the positioning assembly to move the mechanical interface based on the inbound control signal may further comprise moving the mechanical interface based on the received inbound signal. The mechanical interface may be moved from a first position to a second position based on the inbound signal. The method may comprise calculating an amount or angle to move at least one of the first and second motorised joints based on the inbound control signal.

Preferably, the step of controlling the positioning assembly to move the mechanical interface based on the inbound control signal may further comprise determining the position of the mechanical interface before moving the positioning assembly and may further comprise determining the second position for the mechanical position for the mechanical interface based on the inbound control signal and the determined first position.

Alternatively, the step of controlling the positioning assembly to move the mechanical interface based on the inbound control signal may further comprise directly converting the inbound control signal into an amount or an angle to move at least one of the first and second motorised joints.

The inbound control signal may be received from the moveable surgical table. The inbound control signal may be generated by the moveable surgical table and may be transmitted by a transmitter of the moveable surgical table. The inbound control signal may be generated by the surgeon inputting a command to a user interface of the moveable surgical table. The command may be to move the table in a certain way. Alternatively, the inbound control signal may be generated by the surgeon manually repositioning the moveable surgical table. In this latter case, the moveable surgical table may comprise position detectors configured to monitor a change in position of the moveable surgical table. A controller of the moveable surgical table may translate a detected change in position of the moveable surgical table into the inbound control signal.

The inbound control signal may be received from some other transmitter. For example, the transmitter may be part of a telesurgery system allowing communication between the medical robotic arm and a remote surgeon.

The outbound control signal, generated either in response to the mechanical interface being moved by a user of the medical robotic arm or in response to a user input on a user interface of the surgical robotic arm, may be transmitted by a transmitter of the medical robotic arm. The outbound control signal may be operable by the moveable surgical table to move the surgical table.

Preferably, the step of generating an outbound control signal in response to the mechanical interface being moved by a user of the medical robotic arm may comprise calculating the outbound control signal based on positional information of the first and second motorised joints received from the first and second position detectors of the medical robotic arm, respectively. This may comprise determining the first position of the mechanical interface based on positional information of the first and second motorised joints. This may further comprise determining the position of the mechanical interface after it has been moved to the second position based on the positional information. The outbound control signal may be calculated based on the difference between the first position and the second position.

Alternatively, the step of generating an outbound control signal in response to the mechanical interface being moved by a user of the medical robotic arm may comprise determine the amount or angle that each of the first and second motorised joints are moved based on the position of the first and second motorised joints as monitored while the mechanical interface is moved from the first position to the second position. The outbound control signal may be calculated by directly converting the amount or angle of movement of each of the first and second motorised joints into the outbound signal.

According to a fourth aspect of the present disclosure, there is provided a controller for implementing the method according to the third aspect. The controller comprises at least one processor, and memory encoding instructions which, when executed by the at least one processor, cause the at least one processor to be configured to carry out the steps of the method according to the third aspect.

It will be appreciated that the controller of the first, second and fourth aspects may alternatively be implemented in hardware. When implemented in hardware, the controller may be implemented as one or more hardware modules, such as one or more application specific integrated circuits.

The present disclosure further relates to a computer program product comprising software code adapted, when executed on a data processing apparatus, to perform the steps of the method according to the third aspect.

It will be further appreciated that features described in relation to one aspect of the present disclosure may also be applied equally to all of the other aspects of the present disclosure. Features described in relation to the first aspect of the present disclosure may be applied equally to the second and third aspect of the present disclosure, and vice versa. For example, apparatus features of the medical robotic arm described in relation to the first aspect may apply to the medical robotic arm of the system described in relation to the second aspect and/or may be apply to the method described in relation to the third aspect.

### BRIEF DESCRIPTION OF DRAWINGS

The disclosure will be further described, by of example only, with reference to the accompanying drawings, in which:
Figure 1 is a schematic illustration of a medical robotic arm according to a first embodiment;
Figure 2 is a schematic illustration of a system comprising the medical robotic arm of Figure 1 and a moveable surgical table;
Figure 3 is a schematic illustration of the system of Figure 2 after the mechanical interface of the medical robotic arm and the surgical table have been moved from the first position of Figure 2 to a second position;
Figure 4 is flow diagram representing a first method in which the relative position of the surgical table and the mechanical interface of the system of Figure 2 are maintained;
Figure 5 is flow diagram representing a second method in which the relative position of the surgical table and the mechanical interface of the system of Figure 2 are maintained;
Figure 6 is flow diagram representing a third method in which the relative position of the surgical table and the mechanical interface of the system of Figure 2 are maintained;
Figure 7 is flow diagram representing a fourth method in which the relative position of the surgical table and the mechanical interface of the system of Figure 2 are maintained;
Figure 8 is a schematic illustration of the system of Figure 2 in which the surgical table has been titled relative to the position shown in Figure 2;
Figure 9 is a schematic illustration of the system of Figure 2 in which the surgical table has been twisted relative to the position shown in Figure 2;
Figure 10 is a schematic illustration of a medical robotic arm according to a second embodiment; and
Figure 11 is a schematic illustration of a medical robotic arm according to a third embodiment.

### DETAIL DESCRIPTION OF DRAWINGS

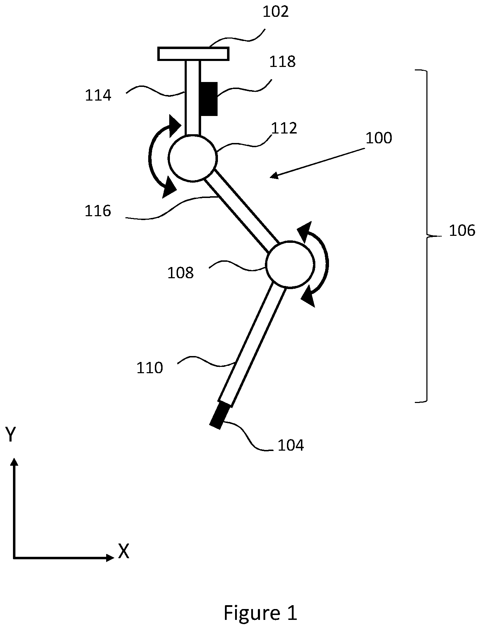

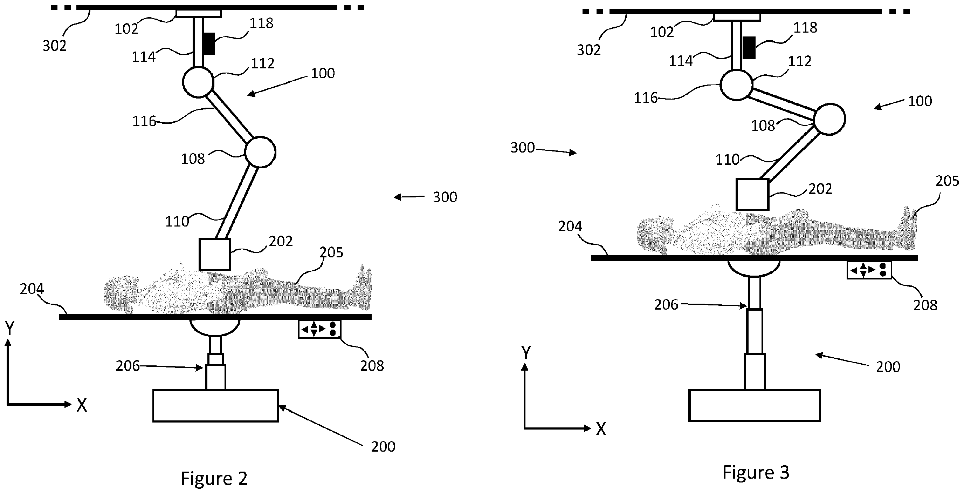

Figure 1 shows a schematic illustration of a medical robotic arm 100. The medical robotic arm comprises an overhead mount 102. The overhead mount 102 is suitable for mounting the robotic arm above a moveable surgical table. In particular, the overhead mount 102 is suitable for mounting the robotic arm to the ceiling 302 above a moveable surgical table 200, as shown in Figure 2. At the opposite end of the moveable robotic arm 100 from the overhead mount 102 is a mechanical interface 104. In Figure 1, the mechanical interface 104 is shown schematically. The mechanical interface 104 is connectable to one or more devices for use in minimally invasive surgery. Each of the devices comprises the same connection means suitable for connecting to the mechanical interface 104. In this way, different devices for use in minimally invasive surgery can be connected to the medical robotic arm 100. Figure 2 shows the medical robotic arm 100 with an attached device 202 which is represented schematically as a square. The device 202 is a torch in this embodiment, but could be any device suitable for minimally invasive surgery.

Connecting the overhead mount 102 to the mechanical interface 104 is a positioning assembly 106. A first end of the positioning assembly 106 is connected to the mechanical interface 104. A second end of the positioning assembly 106 is connected to the overhead mount 102.

The first end of the positioning assembly 106 comprises a first motorised joint 108 which comprises a first stepper motor and a first encoder, not shown in Figure 1. The first end of the positioning assembly also comprises a first arm portion 110, a first end of the first arm portion 110 being connected to the mechanical interface 104 and a second end of the first arm portion 110 being connected to the first motorised joint 108.

The second end of the positioning assembly 106 comprises a second motorised joint 112 which comprises a second stepper motor and a second encoder, not shown in Figure 2. The second end of the positioning assembly also comprises a second arm portion 114, a first end of the second arm portion 114 being connected to the overhead mount 102 and a second end of the second arm portion 114 being connected to the second motorised joint 112.

The first and second encoders are both be single-turn absolute encoders.

A middle portion comprising a middle arm portion 116 connects the first motorised joint 108 to the second motorised joint 112. The first, second and middle arm portions 110, 114 and 116 are rigid.

The first motorised joint 108 acts as a rotatable joint between the first arm portion 110 and the middle arm portion 116. In particular, rotation of the first stepper motor of the first motorised joint 108 causes the first arm portion 110 to move relative to the middle arm portion 116. The second motorised joint 112 acts as a rotatable joint between the second arm portion 114 and the middle arm portion 116. In particular, rotation of the second stepper motor of the second motorised joint 112 causes the second arm portion 114 to move relative to the middle arm portion 116. Because stepper motors are designed to move in a plurality of discrete steps, the position of the arm portions is accurately controllable using the first and second stepper motors. The first and second encoders are configured to monitor the absolute position of the first and second motorised joints 112 and 114.

The first motorised joint 108 is rotatable about an axis that is parallel to the axis of rotation of the second motorised joint 112. In this way, the rotation of the first and/or second motorised joints 108, 112 causes the mechanical interface 104 to move relative to the overhead mount 102 in a plane that is parallel to the axis of rotation. The plane defines an X and Y direction, as shown in Figure 1. The rotation of first and second motorised joints 108, 112 is represented by the curved arrows in Figure 1.

The medical robotic arm further comprises control electronics 118, shown schematically in Figure 1. The control electronics 118 comprise a controller such as a microcontroller, not shown in the Figures. The controller is electrically connected to the first and second stepper motors and the first and second encoders. In this way, the controller can receive signals from the first and second encoder, the signals providing a means for the controller to monitor the absolute position of the first and second stepper motors. As such, the combination of the first encoder and first stepper motor can be referred to as a first position detector configured to monitor the position of the first motorised joint 108. Similarly, the combination of the second encoder and second stepper motor can be referred to as a second position detector configured to monitor the position of the second motorised joint 112.

The controller further comprise a memory in which relevant dimensions of the medical robotic arm 100 are stored. In particular, the dimensions of the first, second and middle arm portions are stored in the memory.

The control electronics 118 further comprise a wireless transceiver configured to send and receive outbound or inbound control signals. The transceiver is a 5G transceiver but could alternatively be a Bluetooth transceiver. The control signals sent and received by the transceiver are encrypted to ensure they are secure. The wireless transceiver is not shown in the Figures.

The control electronics 118 further comprises a user interface, not shown in the Figures. The user interface is connected to the controller. A user such as a surgeon can input commands into the user interface to manipulate the medical robotic arm 100 and, in particular, to reposition the mechanical interface 104. The controller is configured to generate a control signal in response to a user input on the user interface. The controller then controls the positioning assembly 106 to move the mechanical interface 104 according to the control signal.

Figure 2 is a schematic illustration of a system 300 comprising the medical robotic arm 100 of Figure 1 secured to a ceiling 302 by the overhead mount 102 above a moveable surgical table 200. The moveable surgical table 200 comprises a surgical table 204 and a table positioning assembly 206 for moving the surgical table 204. The moveable surgical table 200 further comprises a controller (not shown in the Figures) configured to control the table positioning assembly 206 to maintain the position of the mechanical interface 104 of the medical robotic arm 100 relative to the surgical table 204 based on one or more control signals.

The moveable surgical table 200 comprises a wireless transceiver configured to send and receive outbound or inbound control signals. The transceiver is a 5G transceiver but could alternatively be a Bluetooth transceiver. The control signals sent and received by the transceiver are encrypted to ensure they are secure. The wireless transceiver of the moveable surgical table 200 is not shown in the Figures.

The moveable surgical table 200 comprises position detectors (not shown in the Figures) configured to monitor the position of the surgical table. The position detectors of the moveable surgical table are connected to the controller of the moveable surgical table. The controller of the moveable surgical table 200 is configured to receive signals relating to the position of the surgical table 204. The controller is configured to determine the position of the surgical table 204 based on these signals.

The moveable surgical table 200 further comprises a user interface 208. The user interface 208 is connected to the controller of the moveable surgical table 200. A user such as a surgeon can input commands into the user interface 208 to manipulate the moveable surgical table 200. The controller is configured to generate a control signal in response to a user input on the user interface 208. The controller then controls the table positioning assembly 206 according to the control signal.

A patient 205 is shown lying on the surgical table 204 in Figure 2. The mechanical interface 104 of the medical robotic arm 100 is positioned such that the device 202 is in contact with the patient. The controller of the medical robotic arm 100 and the controller of the moveable surgical table 200 are configured so that this relative positioning of the mechanical interface 104 and the surgical table 204 is maintained automatically following movement of the surgical table 204 and/or the mechanical interface 104 from a first position to a second position.

Figure 3 shows the system 300 of Figure 2 after the surgical table 204 and the mechanical interface 104 have been moved from the first position shown in Figure 2 to the second position shown in Figure 3. As shown in Figures 2 and 3, the first position is lower than the second position but the relative position of the mechanical interface 104 and the surgical table 204 is maintained in both figures. Importantly, the mechanical interface 104 is also in the same position relative to the patient 205 in both first and second positions.

There are several ways in which the position of the mechanical interface 104 and the surgical table 204 can be automatically maintained, as will now be described.

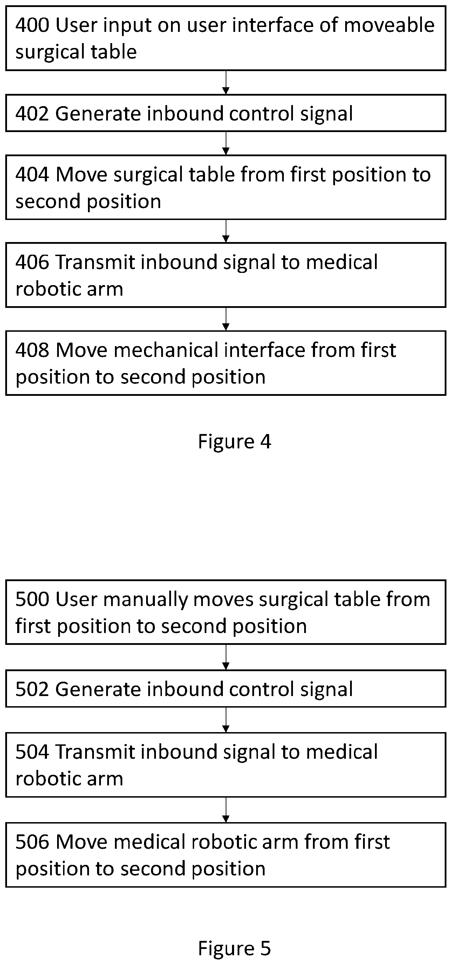

A first method of maintaining the relative position of the surgical table 204 and the mechanical interface 104 is shown in Figure 4.

At step 400, a user inputs a command on the user interface 208 of the moveable surgical table 200.

At step 402, an inbound control signal is generated by the controller of the moveable surgical table 200 based on the user input on the user interface 208. For example, the command on the user interface and the inbound control may both be to move upwards ten millimeters.

At step 404, the controller of the moveable surgical table 200 controls the table positioning assembly 206 to move the surgical table 202 based on the inbound control signal and as commanded by the user. Using the above example, the controller moves the surgical table 204 upwards ten millimeters.

At step 406, the inbound control signal is transmitted from the moveable surgical table 200 by the transceiver of the moveable surgical table 200. The inbound control signal is received by the transceiver of the medical robotic arm 100.

At step 408, the controller of the medical robotic arm 100 controls the positioning assembly 106 to move the mechanical interface from a first position to a second position based on the inbound control signal. This comprises the controller initially determining the position of the mechanical interface based on positional information of the first and second stepper motors received from the first and second encoders. The controller then calculates the number of steps to move each of the first and second stepper motors in order to move the mechanical interface from a first position to a second position. In particular, the controller calculates the amount of rotation of the first and second stepper motors need to move based on the inbound control signal and the lengths of the first, second and middle arm portions 110, 114 and 116 stored in a memory of the controller.

Using the example above, where the control signal includes a command to "move upwards by ten millimeters", the controller calculates the amount of rotation of the first and second stepper motors needed to move the mechanical interface 104 ten millimeters upwards, such that the second position is ten millimeters higher than the first position. In this way, the relative position of the mechanical interface 104 and the moveable surgical table 200 is maintained automatically. In some embodiments, steps 404 and 408 occur simultaneously in such a way that movement of the surgical table 204 is synchronised with movement of the mechanical interface 104. The relative position of the surgical table 204 and mechanical interface 104 then remains substantially constant while moving between the first and second positions.

A second method of maintaining the relative position of the surgical table 204 and the mechanical interface 104 is shown in Figure 5.

At step 500, a user manually moves the surgical table 204 from a first position to a second position. This movement of the surgical table 204 is detected by the controller of the moveable surgical table 200 based on the signals received from the position detectors of the moveable surgical table 200. The controller can the determine the absolute position of the surgical table 204 in both the first position and the second position.

At step 502, the controller of the moveable surgical table 200 generates an inbound control signal. This inbound control signal is based on the difference between the first position and second position of the surgical table, as determined by the controller. For example, if the surgical table 204 is determined to have been moved upwards by ten millimeters between the first and second position, the controller will generate a control signal that includes the command to "move upwards by ten millimeters".

At step 504, the inbound control signal is transmitted from the transceiver of the moveable surgical table 200. The transmitted inbound control signal is then received by the transceiver of the medical robotic arm 100.

At step 506, the controller of the medical robotic arm 100 controls the positioning assembly 106 to move the mechanical interface from a first position to a second position based on the inbound control signal. This comprises the controller initially determining the position of the mechanical interface based on positional information of the first and second stepper motors received from the first and second encoders. The controller then calculates the number of steps to move each of the first and second stepper motors in order to move the mechanical interface from a first position to a second position. In particular, the controller calculates the amount of rotation of the first and second stepper motors need to move based on the inbound control signal and the lengths of the first, second and middle arm portions 110, 114 and 116 stored in a memory of the controller.

Using the example above, where the control signal includes a command to "move upwards by ten millimeters", the controller calculates the amount of rotation of the first and second stepper motors needed to move the mechanical interface 104 ten millimeters upwards, such that the second position is ten millimeters higher than the first position. In this way, the relative position of the mechanical interface 104 and the moveable surgical table 200 is maintained automatically.

In some embodiments, steps 500 to 506 are repeated multiple times while the user moves the surgical table 204. In this case, the "first position" and "second position" do not represent the beginning and end of the motion of the surgical table 204, as moved by the user, but samples of the position of the surgical table 204 during its motion. Reducing the motion into a number of smaller samples of first and second positions and carrying out steps 502 to 506 of the method for each pair of samples means that the medical robotic arm 100 will move the mechanical interface 104 in a way that is synchronise with the motion of the surgical table 204.

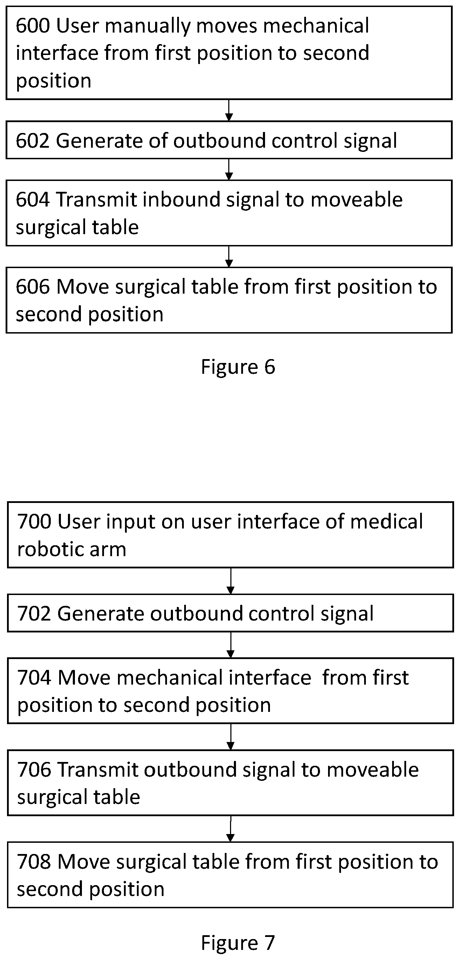

A third method of maintaining the relative position of the surgical table 204 and the mechanical interface 104 is shown in Figure 6.

At step 600, a user manually moves the medical robotic arm 100, and particularly the mechanical interface 104 of the medical robotic arm, from a first position to a second position. This movement is detected by the controller of the medical robotic arm 100 based on the signals received from the first and second position detectors of the medical robotic arm 100. The controller then determines the absolute position of the medical robotic arm 100 in both the first position and the second position.

At step 602, the controller of the medical robotic arm 100 generates an outbound control signal. This outbound control signal is based on the difference between the first position and second position of the mechanical interface 104, as determined by the controller. For example, if the mechanical interface 104 is determined to have been moved upwards by ten millimeters between the first and second position, the controller will generate a control signal that includes the command to "move upwards by ten millimeters".

At step 604, the outbound control signal is transmitted from the transceiver of the medical robotic arm 100. The transmitted inbound control signal is then received by the transceiver of the moveable surgical table 200.

At step 606, the controller of the moveable surgical table 200 controls the table positioning assembly 206 to move the surgical table 204 from a first position to a second position based on the outbound control signal. Using the example above, where the control signal includes a command to move "move upwards by ten millimeters", the controller controls the table positioning assembly 206 to move the surgical table 204 ten millimeters upwards, such that the second position is ten millimeters higher than the first position. In this way, the relative position of the mechanical interface 104 and the moveable surgical table 200 is maintained automatically.

In some embodiments, steps 600 to 606 are repeated multiple times while the user moves the mechanical interface 104. In this case, the "first position" and "second position" do not represent the beginning and end of the motion of the mechanical interface 104, as moved by the user, but are samples of the position of the mechanical interface 104 during its motion. Reducing the motion into a number of smaller samples of first and second positions and carrying out steps 602 to 606 of the method for each pair of position samples means that the medical robotic arm 100 will move the mechanical interface 104 in a more synchronised way with the surgical table 204.

A fourth method of maintaining the relative position of the surgical table 204 and the mechanical interface 104 is shown in Figure 7.

At step 700, a user inputs a command on the user interface of the medical robotic arm to move the mechanical interface 104 as desired.

At step 702, an outbound control signal is generated by the controller of the medical robotic arm 100 based on the user input on the user interface. For example, the command on the user interface and the inbound control may both be to move upwards ten millimeters.

At step 704, the controller of the medical robotic arm controls the positioning assembly 106 to move the mechanical interface 104 based on the outbound control signal and as commanded by the user. This comprises the controller initially determining the position of the mechanical interface based on positional information of the first and second stepper motors received from the first and second encoders. The controller then calculates the number of steps to needed move each of the first and second stepper motors in order to move the mechanical interface from a first position to a second position. In particular, the controller calculates the amount of rotation of the first and second stepper motors that is needed to move the mechanical interface 104 based on the inbound control signal and the lengths of the first, second and middle arm portions 110, 114 and 116 stored in a memory of the controller. Using the above example, the controller moves the mechanical interface upwards ten millimeters.

At step 706, the outbound control signal is transmitted from the medical robotic arm 100 by the transceiver of the medical robotic arm 100. The inbound control signal is received by the transceiver of the moveable surgical table 200.

At step 708, the controller of the moveable surgical table 200 controls the table positioning assembly 206 to move the surgical table 202 based on the outbound control signal. Using the above example, the controller moves the surgical table 204 upwards ten millimeters. In this way, the relative position of the mechanical interface 104 and the moveable surgical table 200 is maintained automatically. In some embodiments, steps 704 and 708 occur simultaneously and in such a way that movement of the surgical table 204 is synchronised with movement of the mechanical interface 104. The relative position of the surgical table 204 and mechanical interface 104 then remains substantially constant while moving between the first and second positions.

Each of the first to fourth methods described above results in the maintenance of the position of the mechanical interface 104 relative to the surgical table 204 based on an inbound or outbound control signal. The system shown in Figure 2 is capable of all four methods of operation. However, in other embodiments, a corresponding system can be provided in which only one or more of the methods is implemented. For example, in one embodiment, the relative position of the mechanical interface 104 and surgical table 204 is only maintained based on user inputs commands on the user interface of the medical robotic arm 100 and/or moveable surgical table 200. Such embodiments do not provide feedback in response to a user manually moving the surgical table or mechanical interface.

Between first position shown in Figure 2 and the second position shown in Figure 3, the surgical table 204 has been moved translationally in the Y axis. The relative position of the mechanical interface 104 and the surgical table 204 can also be maintained if the surgical table 204 is moved translationally in the X axis.

The surgical table 204 is also moveable such that it is tilted or twisted.

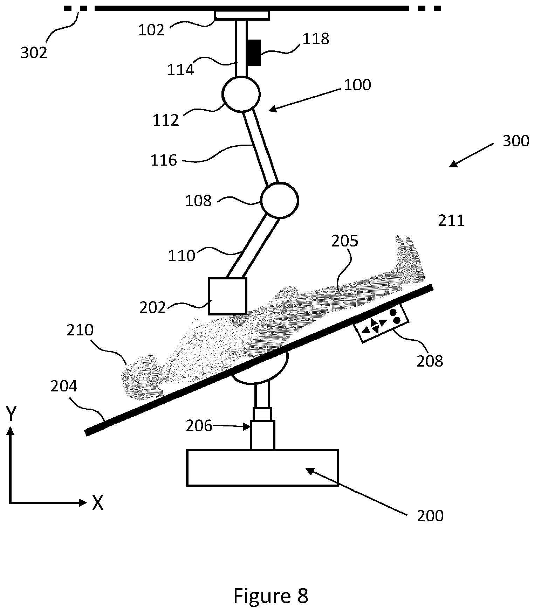

Figure 8 shows the surgical table 204 in a second position which is tilted relative to the first position shown in Figure 2. In such a tilted position, the head 210 of the patient 205 is lowered while the feet 211 are raised. The moveable table is configured to tilt over a range of up to 45 degrees between the two extreme tilt positions.

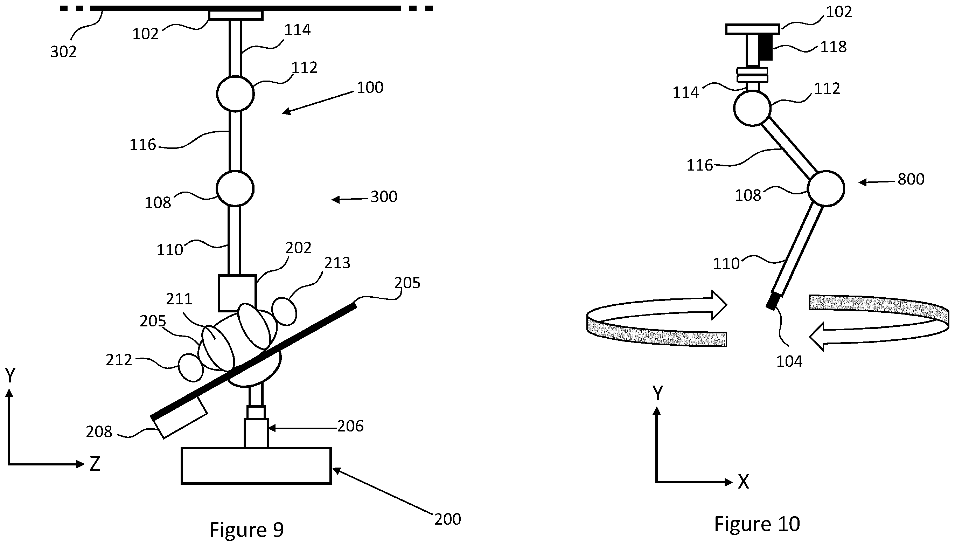

Figure 9 shows the surgical table 204 in a second position which is twisted relative to the first position shown in Figure 2. In such a tilted position, the left hand 212 of the patient 205 is lowered while the right hand 213 is raised. The moveable table is configured to twist over a range of up to 35 degrees between the two extreme twist positions.

In both Figures 8 and 9, the mechanical interface 104 has been moved in order to maintain the position of the mechanical interface 104 relative to the moveable surgical table 204 between the respective first and second positions.

The medical robotic arm 100 described so far is capable of moving the mechanical interface 104 in a plane lying in the X and Y directions. Figure 10 shows a schematic illustration of a second embodiment of a medical robotic arm 800. The medical robotic arm 800 is similar to the first embodiment, and like features have been numbered accordingly. The medical robotic arm 800 further comprises a second positioning assembly 802. The second positioning assembly 802 comprises a rotatable joint having an axis of rotation that is perpendicular to the axis of rotation of the first and second motorised joints 108, 112. This allows for control of the position of the mechanical interface 104 in the Z direction as well as the X and Y direction. This is demonstrated by the arrows in Figure 10.

The medical robotic arm 800 of second embodiment otherwise operates according to the same principle as the first embodiment.

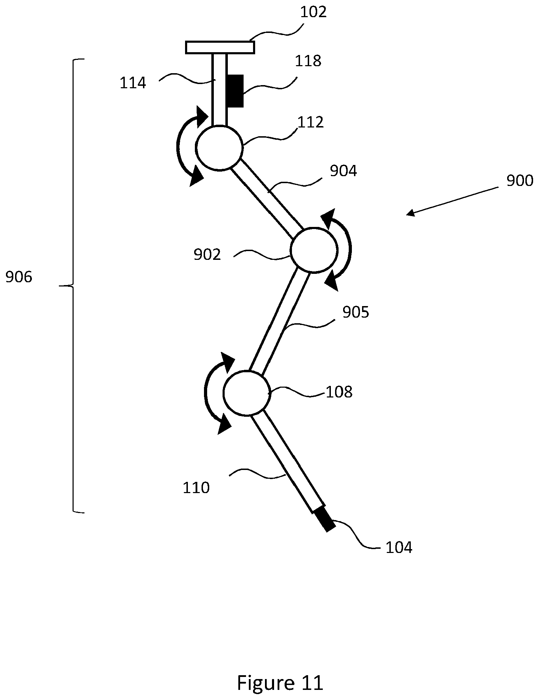

Figure 11 shows a schematic illustration of a third embodiment of a medical robotic arm 900. The medical robotic arm 900 is similar to the first embodiment, and like features have been numbered accordingly. The middle portion of the positioning assembly 906 of Figure 11 additionally comprises a third motorised joint 902 which comprises a third stepper motor and a third encoder. The third encoder is not shown in Figure 11 and is a single-turn absolute encoder. The positioning assembly 906 further comprises a third arm portion 904 and a fourth arm portion 905. A first end of the third arm portion 904 is connected to the first motorised joint 108. A second end of the third arm portion 904 is connected to the third motorised joint 902. A first end of the fourth arm portion 906 is connected to the third motorised joint 902. A second end of the third arm portion 904 is connected to the second motorised joint 112.

The third motorised joint 902 acts as a rotatable joint between the third arm portion 904 and the fourth portion 905. The third motorised joint 902 is rotatable about an axis that is parallel to the axes of rotation of the first and second motorised joint 112. So, like the embodiment of Figure 1, rotation of the first, second and third motorised joints 108, 112, 902 causes the mechanical interface 104 to move relative to the overhead mount 102 in a plane that is parallel to the axis of rotation and that lies in the X and Y direction. The provision of the third motorised joint 902 provides a medical robotic arm with improved agility within the same plane of motion as in Figure 2.

The medical robotic arm 900 of third embodiment operates according to the same principle as the first embodiment.

## Claims

1. A medical robotic arm for use with a moveable surgical table, the medical robotic arm comprising:
an overhead mount for mounting the medical robotic arm above the moveable surgical table;
a mechanical interface connectable to one or more devices for use in minimally invasive surgery;
a positioning assembly comprising a first end comprising a first motorised joint connected to the mechanical interface, a second end comprising a second motorised joint connected to the overhead mount, and a middle portion connecting the first end to the second end;
a first position detector configured to monitor the position of the first motorised joint;
a second position detector configured to monitor the position of the second motorised joint; and
a controller configured to maintain the position of the mechanical interface relative to the moveable surgical table based on one or more control signals.

2. A medical robotic arm according to claim 1, further comprising a receiver for receiving an inbound control signal, the controller being configured to control the positioning assembly to move the mechanical interface based on received inbound control signals, the inbound control signals being related to the position of the moveable surgical table.

3. A medical robotic arm according to claim 2, wherein the receiver is configured for receiving inbound control signals from the moveable surgical table.

4. A medical robotic arm according to claim 2 or 3, wherein the receiver is a wireless receiver.

5. A medical robotic arm according to any one of the preceding claims, further comprising a transmitter for transmitting an outbound control signal operable by the moveable surgical table to move the surgical table.

6. A medical robotic arm according to claim 5, wherein the controller is configured to generate the outbound control signal in response to the mechanical interface being moved by a user of the medical robotic arm from a first position to a second position.

7. A medical robotic arm according to claim 6, wherein the controller is configured to calculate the outbound control signal based on positional information of the first and second motorised joints received from the first and second position detectors respectively.

8. A medical robotic arm according to any of claims 5 to 7, further comprising a user interface connected to the controller for generating a control signal in response to a user input on the user interface, the transmitter being configured to transmit the generated control signal as the outbound control signal and the controller being configured to control the positioning assembly to move the mechanical interface based on the generated control signal.

9. A medical robotic arm according to any one of the preceding claims, wherein the controller is configured to control the positioning assembly to move the mechanical interface based on the control signal operated on by the moveable surgical table.

10. A medical robotic arm according to any one of the preceding claims, wherein the overhead mount is a ceiling mount for mounting the medical robotic arm to a ceiling above the moveable surgical table.

11. A medical robotic arm according to any one of the preceding claims, wherein the first motorised joint comprises a first stepper motor and the first position detector comprises the first motorised joint; and wherein the second motorised joint comprises a second stepper motor and the second position detector comprises the second motorised joint.

12. A medical robotic arm according to any one of the preceding claims, wherein the axis of rotation of the first motorised joint is parallel to the axis of rotation of the second motorised joint.

13. A system comprising the medical robotic arm as defined in any of claim 1 to 12 and a moveable surgical table below the medical robotic arm, the moveable surgical table comprising a surgical table, a table positioning assembly and a controller configured to maintain the position of the mechanical interface of the medical robotic arm relative to the surgical table based on one or more control signals.

14. A method of controlling a medical robotic arm for use with a moveable surgical table, the medical robotic arm comprising an overhead mount for mounting the medical robotic arm above the moveable surgical table; a mechanical interface connectable to one or more devices for use in minimally invasive surgery; and a positioning assembly comprising a first end comprising a first motorised joint connected to the mechanical interface, a second end comprising a second motorised joint connected to the overhead mount, and a middle portion connecting the first end to the second end; the method comprising:
monitoring the position of the first motorised joint with a first position detector and monitoring the position of the second motorised joint with a second position detector; and
maintaining the position of a mechanical interface relative to the moveable surgical table based on one or more control signals.

15. A method according to claim 14, wherein the step of maintaining the position of the mechanical interface relative to the moveable surgical table comprises at least one of the following:
receiving an inbound control signal from the moveable surgical table and controlling the positioning assembly to move the mechanical interface based on the inbound control signal;
generating an outbound control signal in response to the mechanical interface being moved by a user of the medical robotic arm from a first position to a second position and transmitting that outbound control signal to the moveable surgical table; or
generating a control signal in response to a user input on a user interface of the surgical robotic arm; controlling the positioning assembly to move the mechanical interface based on the generated control signal and transmitting the generated signal to the moveable surgical table as an outbound signal.
